# EUROPEAN PATENT APPLICATION

(11) **EP 2 083 012 A1**
(43) Date of publication of application: **29.07.2009**
(21) Application number: 09150968.7
(22) Date of filing: 20.01.2009
(51) Int. Cl.: C07D 513/04, A61K 31/542, A61P 31/04

(54) **1,5-dihydro-2h-pyrrol-2-one tricyclic derivatives having pharmacological activity and processes for their preparation**

(30) Priority: 28.01.2008 IT MI20080129
(71) Applicant: CPC Biotech S.r.l., 80121 Napoli (IT)
(72) Inventor: ARENGHI, Fabio Luigi, 20040, BELLUSCO (MI) (IT)
(74) Representative: Frignoli, Luigi

(57) **Abstract**

Heterocyclic compounds having pharmacological activity, in particular as anti-infectives, structurally correlated with 1,5-dihydro-2H-pyrrol-2-one and processes for their preparation.

## Description

The present invention relates to a class of compounds of formula (I) 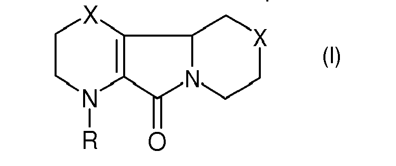 having pharmacological activity, in particular anti-infective, anti-bacterial and/or anti-mycotic activity, in which X is chosen from the group consisting of -S-, -SO-, -SO₂-, -O- and R is a radical chosen from the group consisting of C₁-C₅ alkyls, C₃-C₅ alkenyls, non-substituted C₃-C₇ cycloalkenyls, 4-phenylbenzyl, 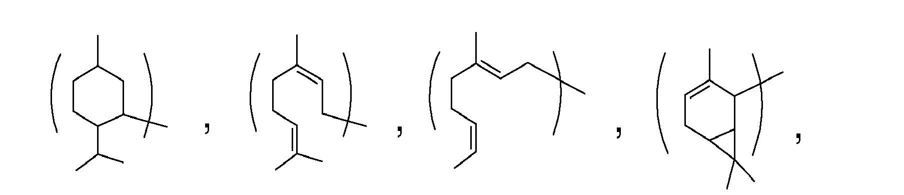 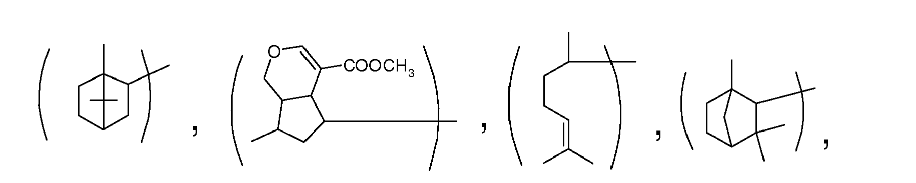 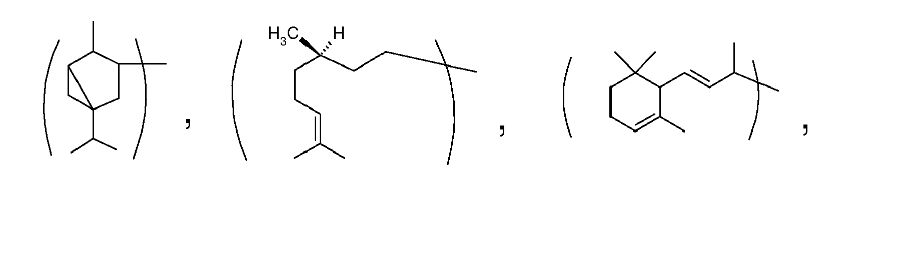 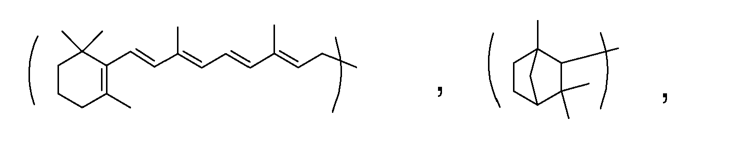 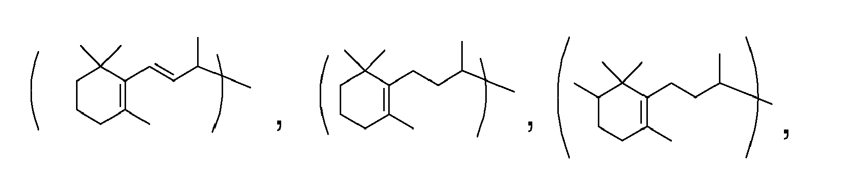 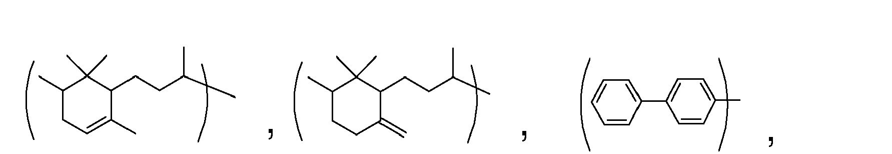 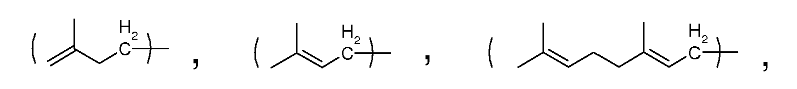 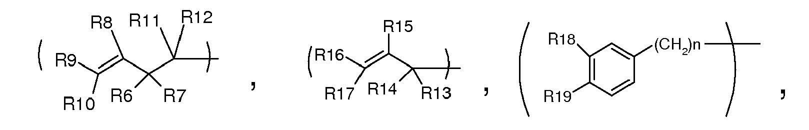 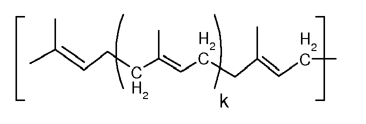 where R₆, R₇, R₈, R₉, R₁₀, R₁₁, R₁₂, R₁₃, R₁₄, R₁₅, R₁₆, R₁₇, are independently chosen from the group consisting of -F, -H, -CH₂F, -CHF₂, - CF₃, -CH₃, C₂-C₅ alkyls, C₂-C₅ alkenyls, C₂-C₅ fluorinated alkyls, C₂-C₅ fluorinated alkenyls, R₁₈, R₁₉ are independently chosen from the group consisting of -H, -F, -Cl, -OH, -OCH₃, -CF₃, n is a whole number equal to 1 or 2, and k is a whole number other than zero.

The compound of formula (Ia) 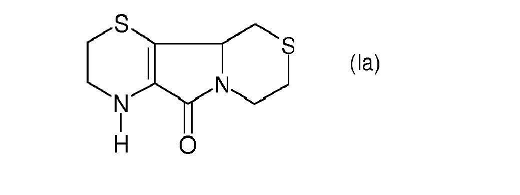 is known as 3,4,7,8,10,10a-hexahydro-2H,5H-pyrrolo[2,1-c:3,4-b']bis[1,4]thiazin-5-one with CAS Registry Number [83923-12-0], and also known as aminoethylcysteine ketimine decarboxylated dimer; it is structurally similar to the compound (I) and is a known secondary metabolite detectable in the plasma and in the urine of healthy subjects, in bovine cephalorachidian liquid, and even in some plants.

The physiological role of said compound (Ia) is not known, and further metabolic modifications have not been detected. Consequently, this is assumed to be a terminal metabolite, excreted as such in urine. Studies have shown that this compound (Ia) possesses strong antioxidant activity, more pronounced than intermediate cysteine metabolites such as taurine and hypotaurine. From in vivo biological studies this known compound has shown considerable antioxidant properties and total non-toxicity even at high doses.

According to the present invention, it has been initially surprisingly verified that by modifying the structure of compound (Ia) by alkylation of the heterocyclic enamino group, new compounds are obtained possessing an unexpected pharmacological activity with anti-infective characteristics.

In particular, according to a first method of alkylating the heterocyclic enamino group present in the structure (Ia), use is made of an excess, up to 5 molar equivalents on the substrate (Ia), of an alkylating species of type R-Y, in aqueous, organic protic or aprotic solvents at temperatures between the freezing and the boiling point of the reaction mass, preferably between -78ºC and the boiling point of the reaction mass obtained, R-being a radical with the same meaning as that expressed in formula (I) and -Y being a nucleofuge, preferably chosen from the group comprising mesylates, tosylates, oxyphosphonium derivatives (such as an oxytrialkylphosphonium or oxytriarylphosphonium), phosphates (such as a dialkylphosphate or a diarylphosphate), alkylsulphenyls, arylsulphenyls, alkylsulphinyls, arylsulphinyls, thiophosphates (such as a dialkylthiophosphate or a diarylthiophosphate), chlorine, bromine, iodine, diazo, diazonium, 2,4,6-trinitroaryloxy. The alkylation reaction can also be conducted using at least 1 molar equivalent on the substrate (Ia) or a base, before adding the alkylating species R-Y to the species (Ia); to effect prior deprotonation of the species (Ia), which can take place at temperatures between the freezing and the boiling point of the reaction mass, preferably between -78ºC and +30ºC, a base is chosen, depending on the reaction conditions, from alkali or alkaline earth metal hydroxides, alkali or alkaline earth metal alcoholates, metal hydrides, alkali metal amides, sodium or lithium hexamethyldisilazide, tetramethylguanidine, guanidine, aliphatic and alicyclic amidines, sodium amide, lithium amide, alkali metal alkyls, alkaline earth metal alkyls, alkali metal aryls, alkaline earth metal aryls, alkyl Grignards, aryl Grignards. Alkylation of the heterocyclic enamino group present in the structure (Ia) can be effected by a different method, according to which a molar excess of the order of 1-5 molar equivalents on (Ia), of the alcoholic species R-OH, in which -R is a radical previously described for formula (I), condenses with the species (Ia); this species R-OH can be advantageously condensed with the species (Ia) in aprotic organic solvents at a temperature between 0ºC and the boiling point of the reaction mass, in the presence of condensing agents preferably chosen from the group comprising N,N'-dicyclohexylcarbodiimide, pentavalent phosphorus halides including in the presence of a N,N-dialkylamide, thionyl chloride, oxalyl chloride, thiophosgene, and tertiary phosphines in the presence of diazo compounds, under the same conditions as the well known Mitsunobu reaction.

The alkylation of the heterocyclic enamino group by means of an alcohol can also be directly effected by using other condensing agents such as 1-ethyl-3-(3-dimethylaminopropyl)carbodiimide in water, in alcohol or in aqueous solvents, including protic, at temperatures between the freezing and the boiling point of the reaction mass, preferably between 0ºC and the boiling point of the reaction mass.

According to the present invention, this synthetic approach, with the two aforesaid N-alkylation methods, has been extended, as a general procedure, to the preparation of all the described structures of formula (I) starting from the corresponding substrates of formula (Ia) and of formula (Ib) 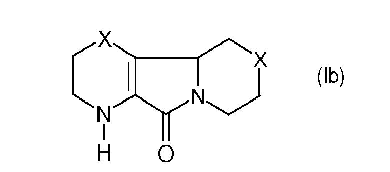 in which the group X is chosen from the group consisting of -SO-, -SO₂-, - O-.

As the products described by the general formula (I) are structurally homologous derivatives of the substrates (Ia) and (Ib), these define a new class of compounds characterized by a virtually homogeneous pharmacological activity.

Different synthetic strategies have been investigated for the formation of substrates of formula (Ia) and (Ib) for alkylation.

The first method enables the synthesis of the compound of formula (Ia) and of the compounds of formula (Ib), where X is -O-, by a general procedure comprising the condensation of a pyruvic acid derivative of formula (II) 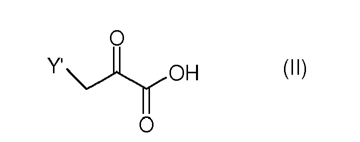 with cysteamine, to obtain the compound (Ia), or with 2-aminoethanol, to obtain the compound (lb) where X is -O-; this condensation reaction is conducted in aqueous protic or aprotic solvents, or in mixtures thereof, in the presence of bases at temperatures between the freezing and the boiling point of the reaction mass, preferably between -78ºC and the boiling point of the reaction mass. The quantity of cysteamine or of 2-aminoethanol used must be at least equimolar to the quantity of the reacting species (II). At the end of condensation the reaction mass is acidified to isolate the product.

The substituent -Y' of the pyruvic acid derivative (II) is a nucleofuge preferably chosen from the group comprising mesylates, tosylates, alkylsulphinyls, arylsulphinyls, alkylsulphonyls, arylsulphonyls, thiophosphates (such as a dialkylthiophosphate or a diarylthiophosphate), chlorine, bromine, iodine, diazo, diazonium, 2,4,6-trinitroaryloxy.

The bases used in the process, in quantities exceeding 3 molar equivalents on the compound (II), are chosen from the group comprising alkali or alkaline earth metal hydroxides, alkali or alkaline earth metal alcoholates, metal hydrides, alkali metal amides, sodium or lithium hexamethyldisilazide, tetramethylguanidine, guanidine, aliphatic and alicyclic amidines, sodium amide, lithium amide, alkali metal alkyls, alkaline earth metal alkyls, alkali metal aryls, alkaline earth metal aryls, alkyl Grignards, aryl Grignards. A variant of the aforedescribed method for synthesizing the compounds (Ia) and (Ib), in which X is -O-, comprises the use of the corresponding alkali or alkaline earth metal thiolates or alcoholates of cysteamine or of 2-aminoethanol in the presence of a base, with molar quantities not less than the corresponding quantity of the reacting species (II), under similar temperature conditions, with the same solvents already used in the condensation between the species (II) and the cysteamine or 2-aminoethanol, ending the process by acidifying the reaction mass to isolate the product.

A further method for synthesizing the compounds (Ia) and (Ib) comprises the autocondensation of 5,6-dihydro-2H-1,4-thiazinyl-3-carboxylic acid to obtain the product (Ia), the autocondensation of 5,6-dihydro-2H-1,4-oxazinyl-3-carboxylic acid to obtain the product (Ib) in which X is -O-, the autocondensation of 5,6-dihydro-2H-1-oxido-1,4-thiazinyl-3-carboxylic acid to obtain the product (Ib) in which X is -SO-, or the autocondensation of 5,6-dihydro-2H-1,1-dioxido-1,4-thiazinyl-3-carboxylic acid to obtain the product (Ib) in which X is -SO₂-.

These reactions take place in the presence of bases at temperatures between the freezing and the boiling point of the reaction mass, preferably between -78ºC and the boiling point of the reaction mass in which the solvents used are aqueous protic or aprotic solvents. The bases are used in molar quantities not less than the value corresponding to the moles of acids used in the autocondensation process: these bases are chosen from alkali or alkaline earth metal hydroxides, alkali or alkaline earth metal alcoholates, metal hydrides, alkali metal amides, sodium or lithium hexamethyldisilazide, tetramethylguanidine, guanidine, aliphatic and alicyclic amidines, sodium amide, lithium amide, alkali metal alkyls, alkaline earth metal alkyls, alkali metal aryls, alkaline earth metal aryls, alkyl Grignards, aryl Grignards; the products obtained, (Ia) and (Ib), are then isolated by acidifying the reaction mass.

The heterocyclic substrates of 5,6-dihydro-2H-1,1-dioxido-1,4-thiazinic type used for the aforesaid autocondensations can be individually synthesized in accordance with general teachings, and the logical equivalents of known cyclization reactions in heterocyclic synthesis, as illustrated for example in "Handbook of Heterocyclic Chemistry" (author: A. R. Katritzky - 1985 - publisher: Pergamon Press), in "Contemporary Heterocyclic Chemistry" (authors: G.R. Newkome and W. W. Paudler - 1982 - publisher: John Wiley & Sons), in "The Chemistry of Heterocycles" (authors: T. Eicher and S. Hauptmann - 2nd edition - publisher: John Wiley & Sons) and in "Chimica Eterociclica" (authors: G.A. Pagani and A. Abbotto - 1995 - publisher: Piccin).

The sulphoxides and sulphones of said heterocyclic substrates can also be produced from the corresponding sulphide. 5,6-dihydro-2H-1,1-dioxido-1,4-thiazinyl-3-carboxylic acid can also be obtained from the corresponding suphoxide. The known methods for oxidizing thioethers to sulphoxides and sulphones comprise the use of organic peracids or hydrogen peroxide, potassium persulphate, potassium permanganate, dimethyldioxirane, periodates, chlorine, sodium hypochlorite, tert-butylhypochlorite, bromine, iodine, sodium perborate and N-aryl-sulphonyloxaziridine. A third synthesis method for the compounds of formula (Ib), in which X is - SO- or -SO₂-, starting from the compound of formula (Ia), comprises the preferable use of the aforesaid reagents useful for oxidizing thioethers to sulphoxides and sulphones; moreover, similar to that already stated, the sulphone (Ib), in which X is -SO₂-, can be obtained from the corresponding sulphoxide (Ib), in which X is -SO-, by using a reagent chosen from those described in the literature which comprise the already mentioned oxidants for transforming thioethers into sulphoxides and sulphones.

As already stated, that described for alkylation of the species (Ia) can be extended to all compounds covered by formula (Ib), which show similar reactivity compared with the already described species R-Y or R-OH. Finally, alkylation of the species described by formulas (Ia) or (Ib), either with the species R-Y or with the species R-OH, can also take place without isolating the compound (Ia) or (Ib) once formed within the reaction environment, using any synthetic strategy applicable from those previously described for the synthesis of said compounds.

For example, certain products included within formula (I) were synthesized in which X is -S-, as alkylated derivatives of the intermediate (Ia), with R chosen from the group comprising methyl (Ii), ethyl (Il), propyl (Im), isopropyl (In, -CH₂-CH=C(CH₃)₂ isoprenyl (Io), -CH₂-Ph benzyl (Ip), -CH₂-CH2-Ph Phenethyl (Iq), -CH₂-CH=C(CH₃)-CH₂- CH₂-CH=C(CH₃)-CH₂-geranyl (Ir) and -Ph-Ph diphenyl (Is): these products have shown antibiotic activity both towards pathogens resistant to β-lactam antibiotics or those characterized by multi-drug resistance, or towards yeasts.

The antimicrobial activity of the substances (Ii), (Il), (Im), (In), (Io), (Ip), (Iq), (Ir), (Is) was evaluated towards 11 clinically isolated Gram-negative and Gram-positive bacterial strains, mostly multi-antibiotic resistant (MDR), and towards two types of yeast of the genus Candida.

A plate method was used (agar spot method or disk diffusion assay - spot quantity 10µl, bacterial inoculum 10⁸ CFU/ml and DMSO negative control): the (Io) and (Ir) concentration used was 100mM. The antimicrobial activity was evaluated by measuring the diameter of the microbial growth inhibition halo expressed in millimetres, the reported values being the average of three experimental points.

The results for the compounds (Io) and (Ir) compared with the known compound (Ia) are shown below

**TABLE A**

| **Microorganism** | **Ia** | **Io** | **Ir** |
|---|---|---|---|
| *Acinetobacter baumannii* M D R | 4 | 11 | 13 |
| *Escherichia coli* | <2 | 12 | 12 |
| *Pseudomonas aeruginosa* M D R | <2 | 11 | 12 |
| *Salmonella* spp. | 3 | 10 | 11 |
| *Klebsiella oxytoca* | - | 8.6 | 9.8 |
| *Klebsiella pneumonite* | - | 10 | 11.5 |
| *Serratia marcescens* | - | 7 | 11 |
| *Enterococcus faecalis* MDR | - | 9 | 13.7 |
| *Enterococcus faecium* M D R | - | 8 | 14.3 |
| *Staphylococcus aureus* M RSA | 4 | 11.4 | 18.6 |
| *Staphylococcus haemolyticus* M D R | <2 | 13 | 15 |
| *Candida albicans* | 6 | 10 | 13 |
| *Candida parapsilosis* | 5 | 8 | 9.8 |

Using scalar quantities of the substances under examination, it was shown that the extent of antibiotic activity is directly proportional to the quantity of substance used. In particular, the molecule (Ir) showed both the best activity characteristics for the widest spectrum, and the strongest activity towards Gram-positive bacteria (*Staphylococcus* spp. and *Enterococcus* spp.).

The minimum inhibitory concentration (MIC) of the substances (Io) and (Ir) towards the methicillin-resistant *Staphylococcus aureus* strain (MRSA) was then evaluated by the method of microdilutions in broth: it should be noted that *Staphylococcus aureus* (MRSA) is one of the main multi-resistant bacteria causing serious problems in hospitals. The measured MICs were 6.25 mmol/I and 1.56 mmol/I for (Io) and (Ir) respectively. From this microbiological evidence it was hence found that the substance (Ir) is characterized by excellent wide spectrum antibiotic activity, even against multi-antibiotic resistant bacteria and against certain yeasts; as (Ir) shows particular effectiveness against staphylococci, often responsible for skin infections, a possible use for it is in the preparation of pharmaceutical formulations for topical use, such as lotions, creams, unguents and ointments.

The ensuing proposed experimental examples do not limit the invention.

### EXAMPLE 1

### Preparation of 3,4,7,8,10,10a-hexahydro-2H,5H-pyrrolo[2,1-c:3,4-b']bis[1,4]thiazin-5-one, compound (Ia).

3.34 g (20 mmol) of 3-bromopyruvic acid are dissolved in 5 ml of water and 2.27 g (20 mmol) of cysteamine hydrochloride are added after dissolution in a solution consisting of 10 ml of 6N aqueous sodium hydroxide and 1 ml of water.

After 30 minutes of agitation 4 ml of 6N hydrochloric acid are added and the solution cooled to a temperature between 0ºC and +5ºC, the formation of a pale yellow precipitate being noted after a further 30 minutes. The precipitate is filtered off and suspended in 120 ml of water. Said suspension is heated to boiling and agitated for 30 minutes, after which it is cooled to +4ºC and left to crystallize at that temperature for 12 hours. The precipitate is filtered off and washed with a total of 10 ml of water in two portions. The product is finally dried under reduced pressure to obtain 1.15 g of the compound (la).
Melting point: +142ºC.
ε (308 nm): 6000 l/(mol.cm)
FT-IR: 3390 cm⁻¹, 1730 cm⁻¹, 1685 cm⁻¹, 1640 cm⁻¹.
Mass spectroscopy (GC) with fragmentation: (m/z) 228, 213, 200, 191, 181, 167, 154, 140, 126, 112, 99, 90, 80, 71, 54.

### EXAMPLE 2

### Preparation of the compounds (Ii), (Il), (Im), (In), (Io), (Ip), (Iq), (Ir), (Is).

The aforestated compounds were synthesized starting from the product (Ia) dissolved in isopropanol by adding an excess of the appropriate alkyl, benzyl or aryl bromide, to the extent of 3-5 molar equivalents on (la). The reaction mixture is brought to a temperature of +40ºC and left to react for 4 hours, or to a temperature of +30ºC and left to react for 24 hours.

On termination of the reaction the chosen product (Ii), (Il), (Im), (In), (Io), (Ip), (Iq), (Ir) or (Is) precipitates on cooling. The precipitate is filtered off and washed with ethanol, then dried under reduced pressure. All nine products obtained in this manner showed purities, determined by gas chromatography/mass spectroscopy (GC/MS), exceeding 95%. The yields for each product starting from (Ia), with the appropriate alkylating agent used, are shown in the following

**TABLE B**

| Substrate | Reagent | Product | Molar yield |
|---|---|---|---|
| (Ia) | methylbromide | (Ii) | 75 % |
| (Ia) | ethylbromide | (Il) | 70 % |
| (Ia) | propylbromide | (Im) | 73 % |
| (Ia) | isopropylbromide | (In) | 71 % |
| (Ia) | 1-bromo-3-methylbut-2-ene (dimethylallylbromide) | (Io) | 72% |
| (Ia) | benzylbromide | (Ip) | 74 % |
| (Ia) | 1-bromo-2-phenylethane (phenylethylbromide) | (Iq) | 45 % |
| (Ia) | (2E)-1-bromo-3,7-dimethylocta-2,6-diene (geranylbromide) | (Ir) | 77 % |
| (Ia) | 1-bromo-4-phenylbenzene | (Is) | 73 % |

Mass spectroscopy (GC) with fragmentation for the compound (Ir): (m/z) 364, 307, 281, 228, 200, 182, 154, 126, 99, 84, 69, 41.

### EXAMPLE 3

### Preparation of the compound (Io).

### (lo) is the compound of formula (I) where X is -S- and R is -CH₂-CH=C(CH₃)₂.

119 mg (0.5 mmol) of (Ia) are dissolved in 1.25 ml of a 1.5/1 (v/v) isopropanol/2-methoxyethanol mixture, then 0.180 ml of dimethylallylbromide (1.5 mmol) are added. The solution is agitated for 48 hours at ambient temperature until the formation of a brown precipitate is noted, this then being filtered off, washed with a little ethanol and dried under reduced pressure. 140 mg of the compound (Io) are obtained.

Mass spectroscopy (GC) with fragmentation for the compound (Io): (m/z) 296, 281, 253, 241, 228, 213, 200, 182, 167, 154, 140, 126, 112, 99, 85, 69,53,41.

## Claims

1. Compounds of formula (I) 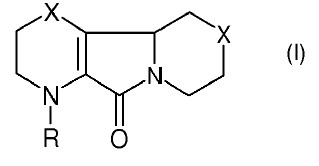 having pharmacological activity, in particular anti-infective, anti-bacterial and/or anti-mycotic activity, wherein X is chosen from the group consisting of -S-, -SO-, -SO₂-, -O- and R is a radical chosen from the group consisting of C₁-C₅ alkyls, C₃-C₅ alkenyls, non-substituted C₃-C₇ cycloalkenyls, 4-phenylbenzyl, 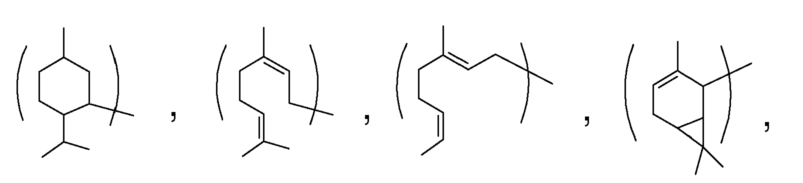 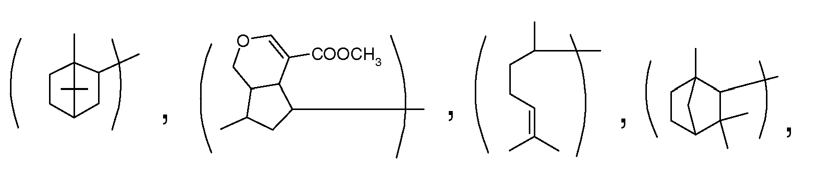 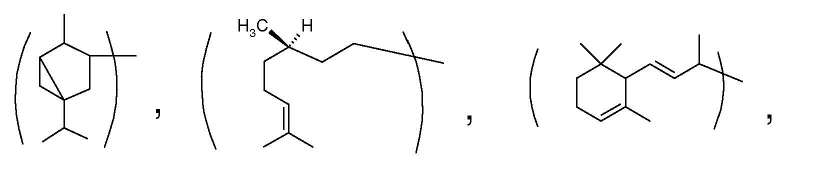 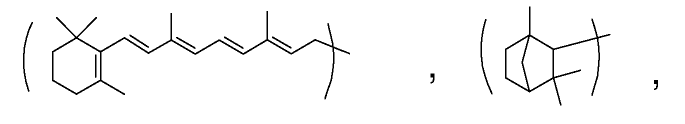 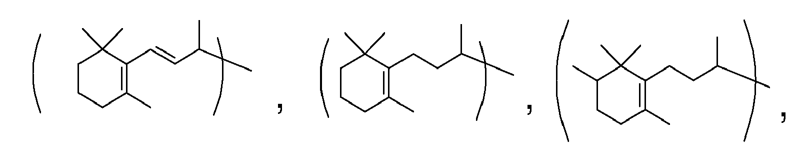 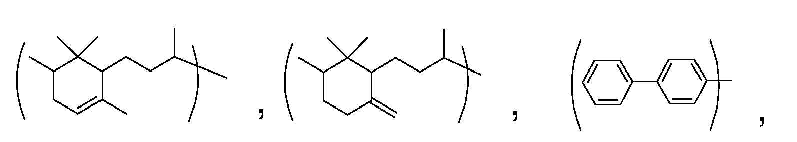 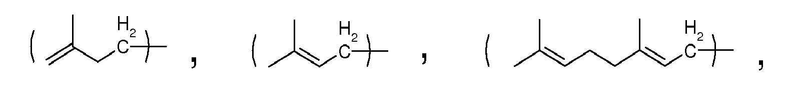 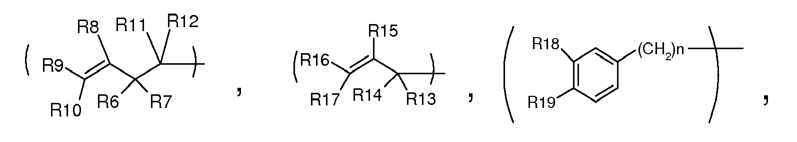 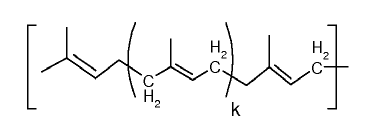 where R₆, R₇, R₈, R₉, R₁₀, R₁₁, R₁₂, R₁₃, R₁₄, R₁₅, R₁₆, R₁₇, are independently chosen from the group consisting of -F, -H, -CH₂F, -CHF₂, - CF₃, -CH₃, C₂-C₅ alkyls, C₂-C₅ alkenyls, C₂-C₅ fluorinated alkyls, C₂-C₅ fluorinated alkenyls, R₁₈, R₁₉ are independently chosen from the group consisting of -H, -F, -Cl, -OH, -OCH₃, -CF₃, n is a whole number equal to 1 or 2, and k is a whole number other than zero.

2. A process for preparing a compound according to claim 1, wherein the compound of formula (la) 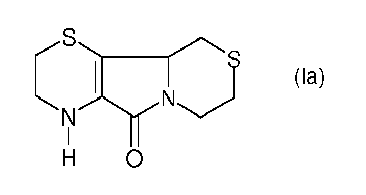 or a compound of formula (Ib) 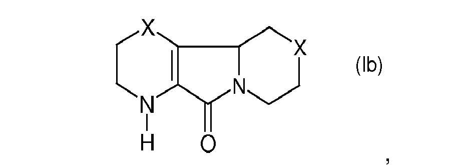 in which X is chosen from the group consisting of -SO-, -SO₂- and -O-, is reacted with an alkylating species of type R-Y, R- being a radical with the same meaning as that expressed in formula (I) of claim 1 and -Y being a nucleofuge.

3. A process for preparing a compound according to claim 1, wherein the compound of formula (Ia) 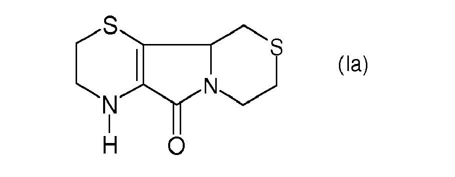 or a compound of formula (Ib) 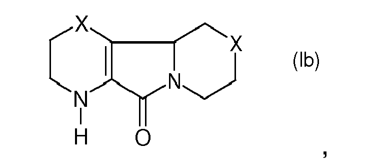 in which X is chosen from the group consisting of -SO-, -SO₂- and -O-, is reacted with the alcoholic species R-OH, R- being a radical with the same meaning as that expressed in formula (I) of claim 1.

4. A process for preparing the compound of formula (Ia) or (Ib) 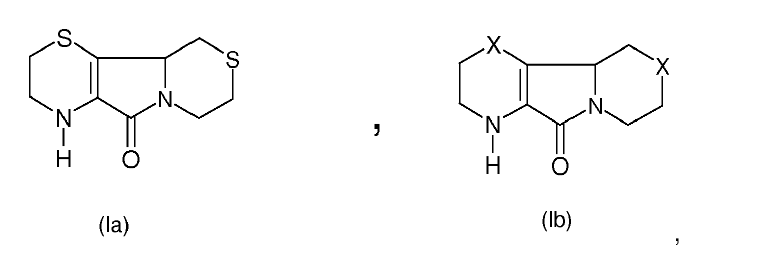 in which X is -O-, by reacting a pyruvic acid derivative of formula (II) 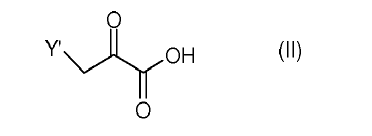 in which the substituent -Y' is a nucleofuge group, with cysteamine to obtain the compound of formula (Ia), or with 2-aminoethanol to obtain the compound of formula (Ib) or with alkali or alkaline earth metal thiolates of cysteamine to obtain the compound of formula (Ia), or alkali or alkaline earth metal alcoholates of 2-aminoethanol to obtain the compound of formula (Ib).

5. A process for preparing a compound of formula (Ia) or (Ib) 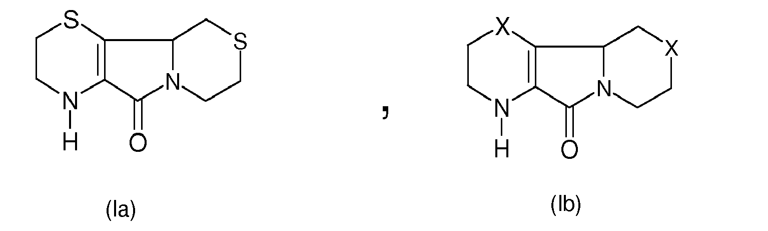 in which X is chosen from the group consisting of -SO-, -SO₂- and -O-, by the autocondensation of 5,6-dihydro-2H-1,4-thiazinyl-3-carboxylic acid to obtain the compound of formula (Ia), the autocondensation of 5,6-dihydro-2H-1,4-oxazinyl-3-carboxylic acid to obtain the compound of formula (Ib) in which X is -O-, the autocondensation of 5,6-dihydro-2H-1-oxido-1,4-thiazinyl-3-carboxylic acid to obtain the compound of formula (Ib) in which X is -SO-, or the autocondensation of 5,6-dihydro-2H-1,1-dioxido-1,4-thiazinyl-3-carboxylic acid to obtain the compound of formula (Ib) in which X is -SO₂-.

6. A process for preparing a compound of formula (Ib) 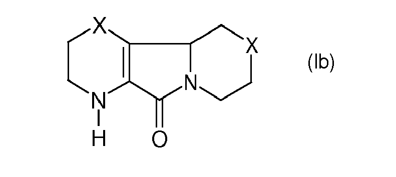 in which X is chosen from the group consisting of -SO- and -SO₂-, comprising oxidizing the compound of formula (Ia)

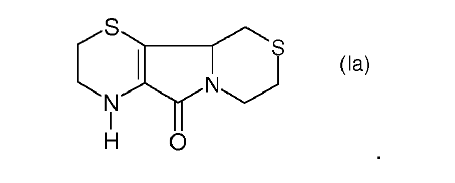

7. Compounds of formula (Ib) 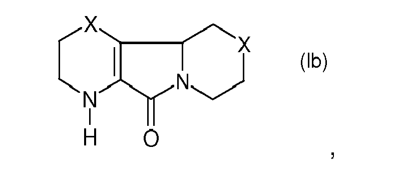 in which X is chosen from the group consisting of -SO-, -SO₂- and -O-.

8. Use of a compound according to claim 1 for preparing an anti-infective medicament.

9. Use as claimed in claim 8, wherein said medicament is active against fungi and yeasts.

10. Use as claimed in claim 8, wherein said medicament is active against gram positive and gram negative bacteria.
